# EUROPEAN PATENT APPLICATION

(11) **EP 1 219 313 A1**
(43) Date of publication of application: **03.07.2002**
(21) Application number: 00311773.6
(22) Date of filing: 29.12.2000
(51) Int. Cl.: A61M 11/00, A61M 15/00, A61M 16/20, A61M 16/18

(54) **Liquid discharging apparatus and magneto-shape-memory type valve**

(71) Applicant: INSTRUMENTARIUM CORPORATION, 00510 Helsinki (FI)
(72) Inventor: Haveri, Heikki, 00360 Helsinki (FI)
(74) Representative: Charlton, Peter John

(57) **Abstract**

A liquid discharge apparatus, such as a nebulizer discharges liquid droplets by supplying the liquid to a vibrating element having a plurality of openings. A valve between a reservoir for the liquid and the element has a flexible tube extending toward the element. The tube extends through the magnetic field of a magnetic coil. A stopper in the tube is formed of magneto-shape-memory material. Energization of the magnetic coil establishes a flow channel configuration in the stopper by distorting the stopper to allow liquid to flow to the vibrating element. The apparatus may include an electrode for measuring, in conjunction with the vibrating clement, an impedance indicative of the amount of liquid provided to the element. The impedance so measured may be used to control the supply of liquid from the reservoir. In another embodiment of the valve, the valve is placed in the reservoir and an actuator formed of magneto-shape-memory material located outside the tube is used to operate the valve.

## Description

### BACKGROUND OF THE INVENTION

The present invention relates to an improved valve structure and to liquid discharge apparatus, such as a nebulizer apparatus containing same. Nebulizers, or atomizers, are devices that generate a fine spray or aerosol, usually of liquid. A particularly useful application for nebulizers is to provide a fine spray containing a dissolved or a suspended particulate or colloidal pharmaceutical agent for administration to a subject by inhalation. Such inhalation treatment is highly effective for conditions affecting the subject's respiratory organs. Further, since the lungs are close to the heart and the blood circulatory system of the body, drug administration by inhalation provides an effective and rapid delivery system to all organs of the body. Other applications for liquid discharge apparatus include dispensing insecticides, paint or other coatings, deodorants, water for humidification, etc. Other devices that may incorporate the present invention include printers in which ink is discharged onto paper.

When dispensing a pharmaceutical agent, the nebulizer may be placed directly in the mouth or nose of the subject so that the spray can be entrained in the respiratory gases inhaled during normal, spontaneous breathing of the subject. In other cases, the subject breathes with the aid of a respiratory ventilator. A typical ventilator has a breathing circuit comprising an inhalation limb and an exhalation limb connected to two arms of a Y-connector. The third arm of the Y-connector is connected, via a patient limb, to a mouthpiece, mask or endotracheal tube for the subject. The ventilator provides a complete or partial supply of respiratory gases to the subject through the inhalation limb during inhalation. The contraction of the subject's lungs discharges gas through the exhalation limb during exhalation. When a nebulizer is employed in conjunction with a ventilator, it is typically placed in the patient limb but can also be placed in the inhalation limb.

Nebulizers currently in use for ventilator applications generate the spray either pneumatically or by means of ultrasonic vibrations. Pneumatic nebulizers are typically used with a liquid, such as an aqueous drug solution. High pressure driving gas is conducted through a nozzle to draw the drug from a drug supply for the nebulizer. The drug is discharged against a baffle or other means in a gas space of the nebulizer, breaking the liquid into a fine spray. The gas space is in fluid communication with the inhaled gas pathway of the breathing circuit so that the gas flow discharged from the nozzle along with the nebulized drug is conducted to the breathing circuit and ultimately to the subject.

Disadvantages in the use of pneumatic nebulizers include the following. If the nebulizer adds a significant quantity of gas, for example, up to five liters/minute, into the breathing circuit, the breathing gas composition may be affected. Due to passage of the driving gas through the nozzle, impingement of the drug on the baffle, etc., pneumatic nebulizers are noisy. And, controlling the commencing and stopping of a drug agent spray is difficult and is not very accurate, resulting in wastage of the drug.

The foregoing shortcomings of pneumatic nebulizers have led to the use of ultrasonic nebulizers in which the fine spray is produced by ultrasonic vibration of the liquid, as by a piezoelectric crystal. Breathing gas composition and the on-off operation are easier to control with such nebulizers than in a pneumatic nebulizer. However, existing ultrasonic devices may require a large, bulky electrical power supply to power the crystal and may not be able to nebulize colloidal or particulate suspensions.

In one type of ultrasonic nebulizer, the fine spray is produced by dropping the liquid on, or otherwise applying it to, the vibrating element. See U.S. Patent 5,443,059. U.S. Patent 3,812,854 describes another type of nebulizer for inhalation therapy in which the spray is generated on the front surface of a vibrating, porous body. The pores of the body form a network of passages that enable the liquid to flow through the body. The liquid to be nebulized is supplied under pressure from a liquid supply through a liquid conduit to the pores, and forced through the pores to the front surface of the porous body where it is discharged as a spray. U.S. Patent 5,487,378 describes a nebulizer in which the aerosol is formed using a mesh plate instead of a porous solid body. The mesh plate has a plurality of orifices for the liquid. The liquid or the nozzle assembly is vibrated ultrasonically by a piezoelectric element to nebulize a dose of liquid as it passes through the mesh plate.

U.S. Patents 5,518,179 and 5,299,739 describe a nebulizer in which capillary feed is used to supply liquid to the vibrating element. A further alternative for liquid supply is achieved by condensing a liquid vapor on one face of a membrane, the liquid thus condensed being dispensed in droplet form. See U.S. Patent 5,518,179.

U.S. Patent 5,938, 117 describes an apparatus for dispensing liquids as an atomized spray having a fluid supply system that transports liquid to an apertured oscillating surface. The fluid supply system is connected to an electronic flow control valve. The valve is connected to an electronic circuit. In the event of excessive delivery of liquid, the oscillation amplitude decreases and the current draw by the piezoelectric element decreases. A current sensor circuit senses the current draw and transmits an overflow signal to the flow control valve to reduce the delivery rate of liquid to the surface until the amount of fluid returns to a normal level.

A specific difficulty with current liquid discharge apparatus, such as nebulizers, lies in the liquid supply system containing a valve that supplies liquid to the vibratable element for atomization. In the past such valves have been difficult to control or have consumed large amounts of power. Also, some parts of the nebulizer may come into contact with the breathing gas of the patient and those parts should be carefully cleaned or changed to minimize infections when changing between patients. Other parts of the nebulizer may furthermore be in contact with different anaesthetic fluids, which are specific to each patient. Those parts should also be cleaned or changed when changing to a different patient. Still other parts of the nebulizer, including parts of the liquid supply system, are in contact with the drug administered to a given subject. These parts should be cleaned when changing to a different drug or a different subject.

The foregoing problems have increased the cost and complexity and reduced the ease of use of existing liquid discharge apparatus.

### BRIEF SUMMARY OF THE INVENTION

An object of the present invention is to provide an improved valve and liquid discharge apparatus having a liquid supply system containing such an improved valve for supplying liquid to a displaceable element so that the liquid can be discharged from the apparatus. Another object of the invention is to provide an apparatus having such a liquid supply system in which those parts that are exposed to the liquid during operation are changeable or/and disposable to reduce the cleaning work when changing liquids or, if the apparatus is a drug dispensing nebulizer, when changing subjects.

The above objects are attained by a liquid discharge apparatus comprising displaceable element such as a vibrating mesh plate. A liquid supply system includes the valve of the present invention which has a fixed part and a changeable or disposable part to control liquid flow to the displaceable element. The liquid supply system has a liquid reservoir attached to the changeable part of the valve from which the liquid is fed through the valve. A liquid detection system detects the liquid arriving at the displaceable element from the fluid reservoir and controls the valve which regulates the liquid flow.

The control valve of the present invention comprises a magnetic field source for selectively forming a magnetic field. The magnetic field source comprises a fixed part of the valve. In one embodiment of the valve, an elastic tube through which fluid may flow lies in the magnetic field of the magnetic field source, when formed. A stopper in the tube is formed of a material which changes from a residual shape to a deformed shape when subjected to a magnetic field. The stopper has a flow channel configuration which is sealed by the tube when the stopper is in one of the residual shape or deformed shape to prevent fluid flow through the tube. The flow channel configuration is opened to form a fluid flow path in the tube when the stopper is placed in the other of the deformed shape or residual shape by subjection to the magnetic field of the magnetic field source. When used in the liquid discharge apparatus of the present invention, the valve is typically closed when in the residual shape and is opened when the magnetic field of the magnetic field source is formed.

In another embodiment of the invention, a valve is placed in the reservoir and operated by an actuator located outside the tube through which the liquid flows from the reservoir to the displaceable element.

Various other features, objects, and advantages of the invention will be made apparent from the following detailed description and the drawings.

### BRIEF DESCRIPTION OF THE SEVERAL VIEWS OF THE DRAWING

The foregoing objects and advantages, as well as the invention itself, will be more fully understood from the attached drawing and following detailed description.

In the drawings:
Fig. 1 is a general cross section view of a liquid discharge apparatus of the present invention including a liquid supply controlling valve, the operating environment for the apparatus being shown in generalized schematic form;
Fig. 2 is an exploded, cross sectional view showing the apparatus of the present invention;
Figs. 3a and 3b are cross sectional views showing filling of the liquid reservoir of the apparatus of Fig. 1;
Figs. 4a and 4b are schematic views showing operation of the displaceable means of the apparatus of Fig. 1;

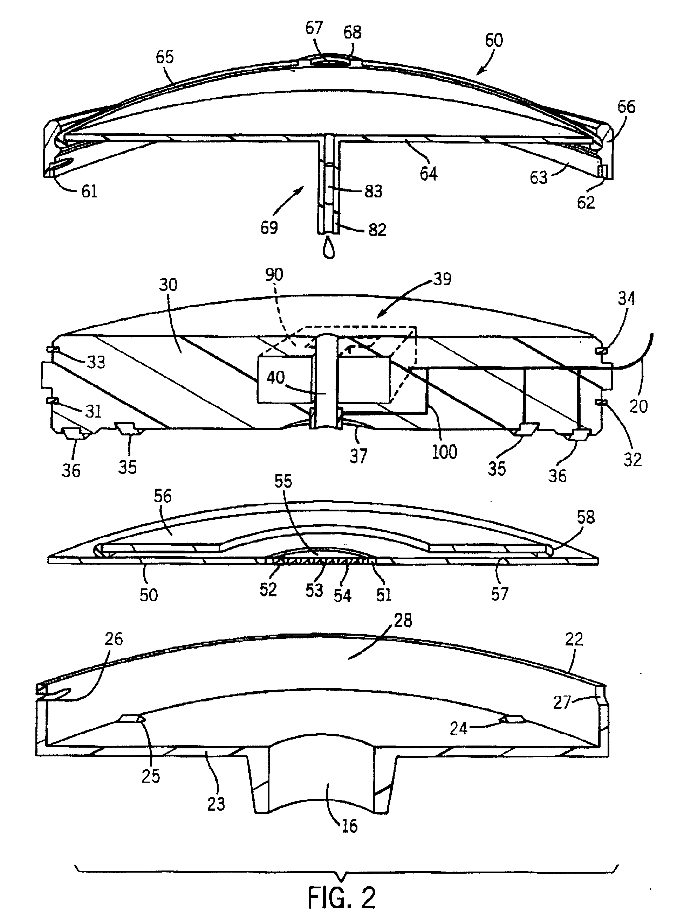

Figs. 5a-1 and 5a-2 are generally orthogonal views of one embodiment of the part of the valve shown in Fig. 2, that controls liquid flow from the reservoir, said part being in the changeable or disposable part of the valve;
Figs. 5a-3 and 5a-4 are generally orthogonal views of another embodiment of the part of the valve that controls liquid flow from the reservoir;

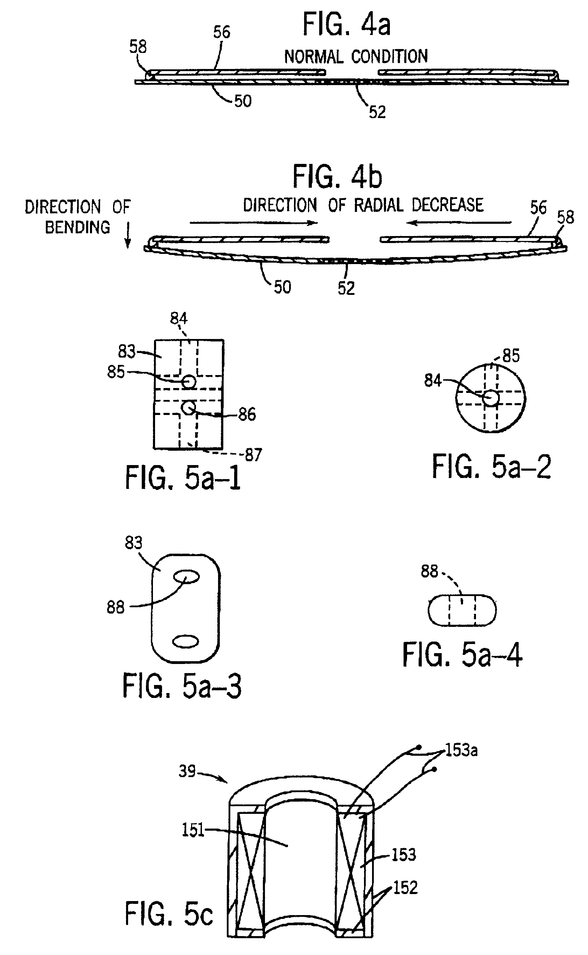

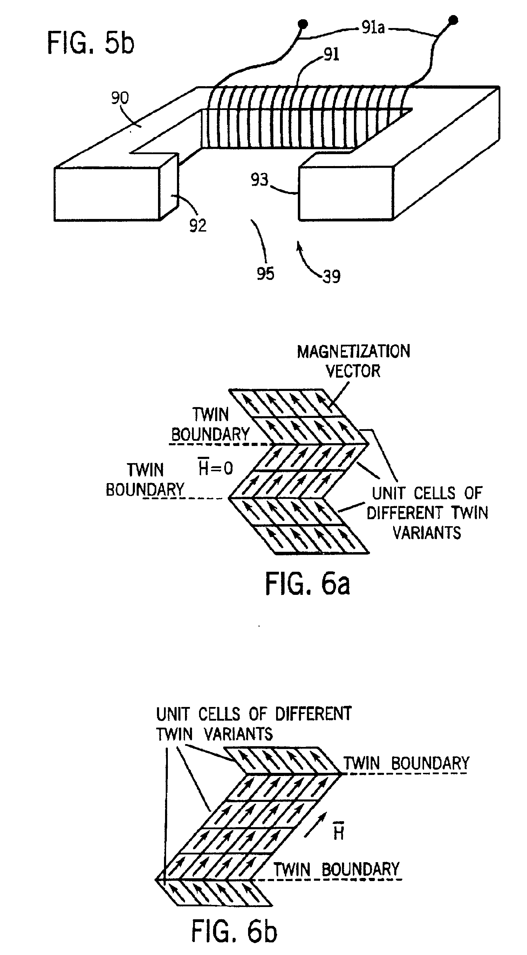

Figs. 5b and 5c show magnetic field coils forming a fixed part of the valve;
Figs. 6a and 6b are schematic views of a material that changes shape in a magnetic field and used to form the parts shown in Fig. 5a, the views showing the turning of the unit cells by a magnetic field;

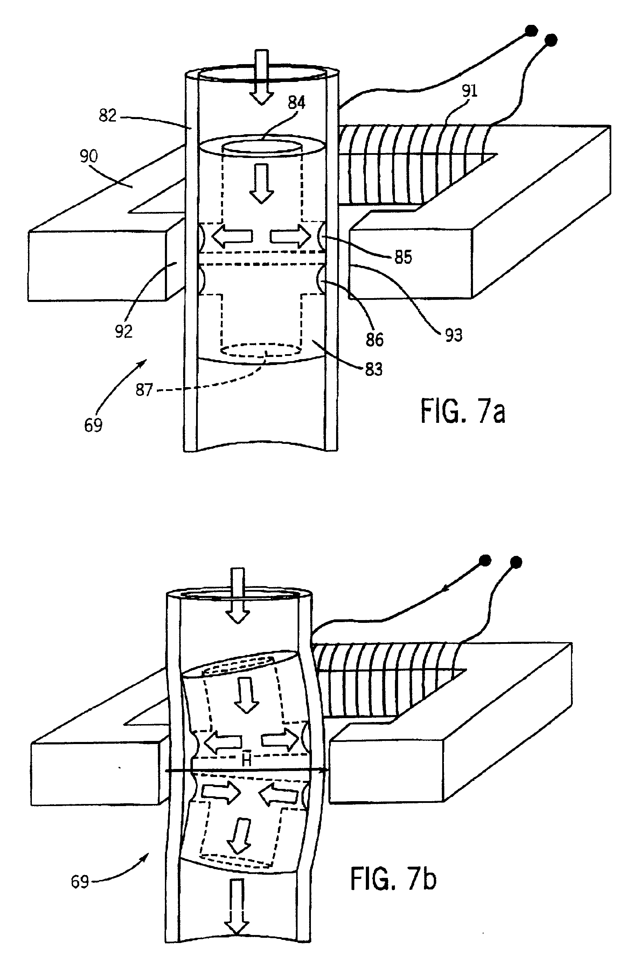

Figs. 7a and 7b are cross-sectional schematic views showing functioning of the valve using the part shown in Figs. 5a-1 and 5a-2;

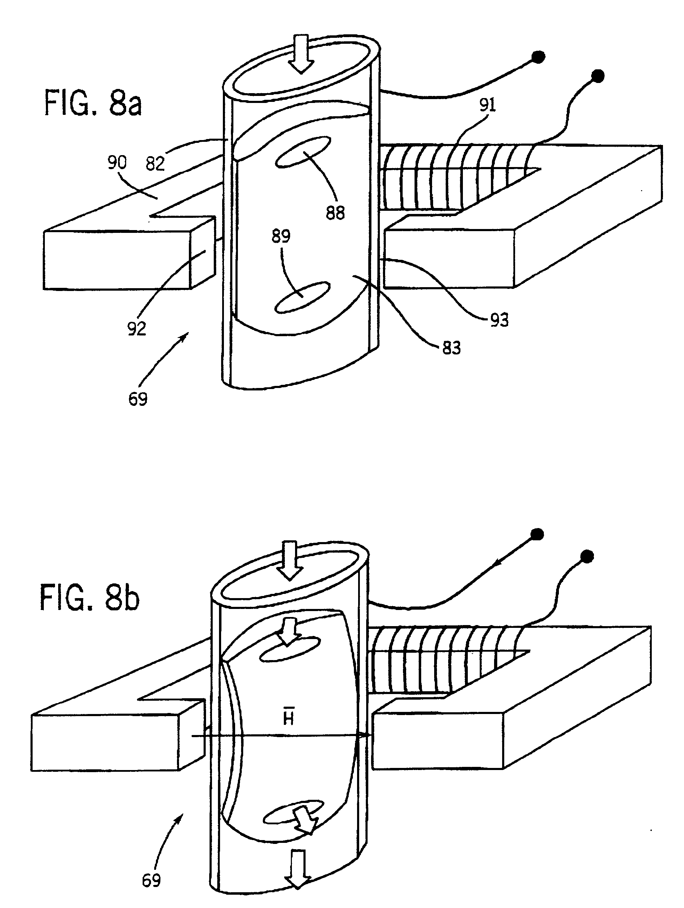

Figs. 8a and 8b are cross-sectional schematic views showing functioning of the valve using the part shown in Figs. 5a-3 and 5a-4;

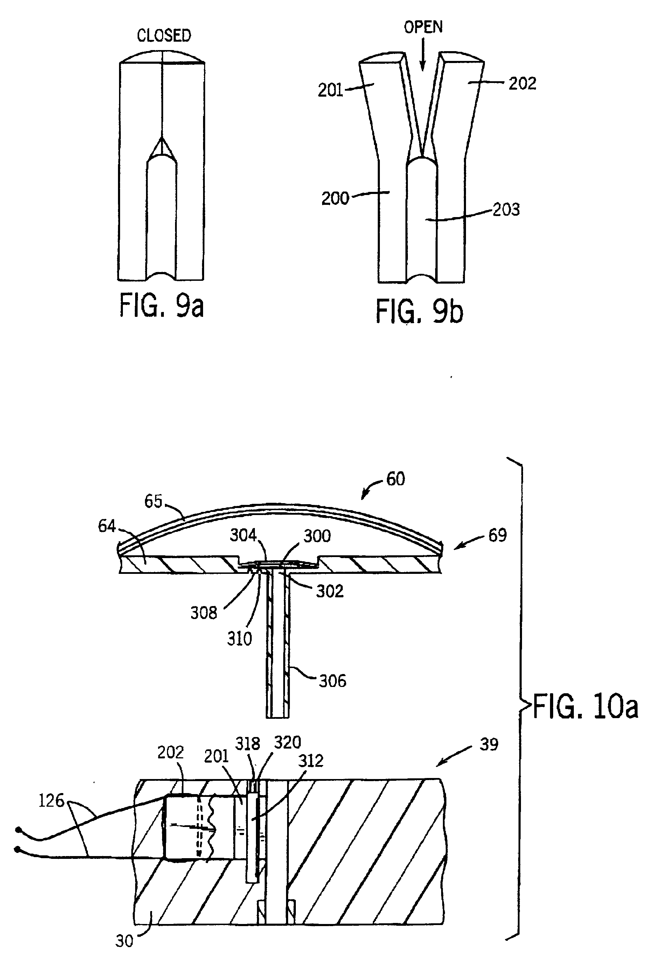

Figs. 9a and 9b show another embodiment of the part of the valve shown in Fig. 2 that controls liquid flow from the reservoir;

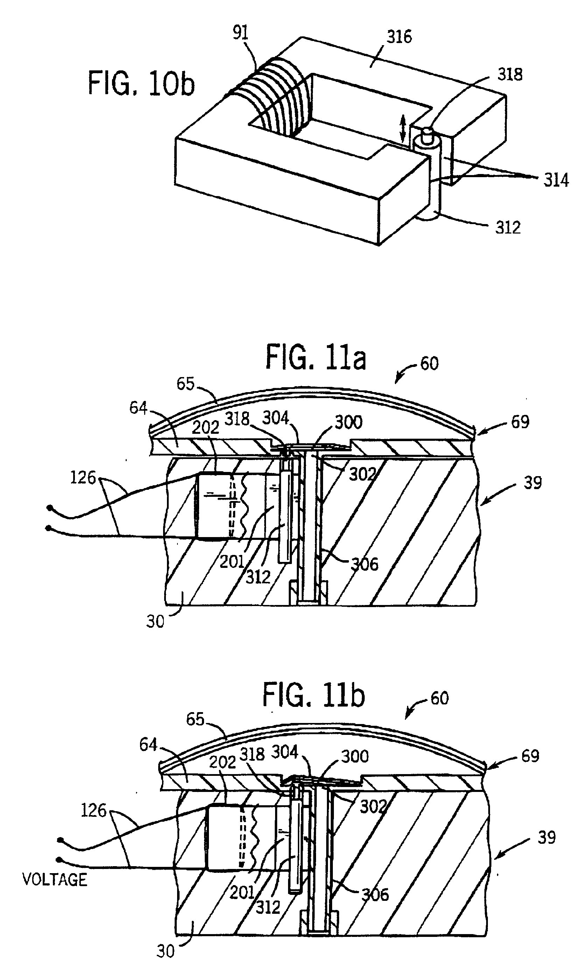

Figs. 10a and 10b show a further embodiment of the valve of the present invention; and
Figs. 11a and 11b illustrate operation of the valve shown in Fig. 10.

### DETAILED DESCRIPTION OF THE INVENTION

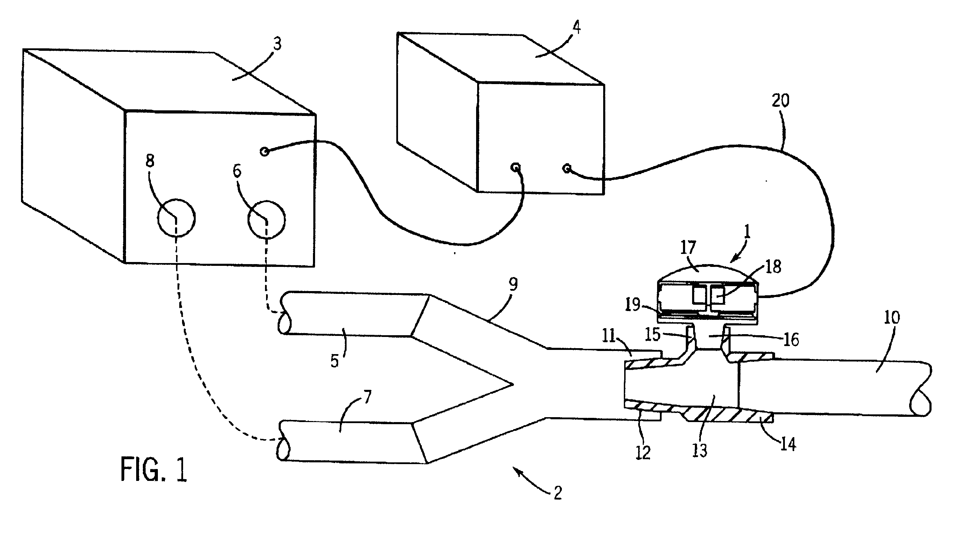

Liquid discharge apparatus 1 of the present invention is shown as a nebulizer in Fig. 1 and is typically used in conjunction with breathing circuit 2, ventilator 3 and control unit 4. The nebulizer 1 atomizes liquid solutions or suspensions for delivery to a subject, as for example as a drug treatment for a patient. Breathing circuit 2 includes inhalation limb 5, which is coupled to ventilator 3 at inhalation limb connector 6. Exhalation limb 7 is connected to ventilator 3 at exhalation limb connector 8. Inhalation limb 5 and exhalation limb 7 are connected to two arms of Y-connector 9. A third arm of Y-connector 9 is connected to one end of patient limb 10. The other end of patient limb 10 is directed to a mouthpiece, facemask, or endotracheal tube for the subject.

Ventilator 3 provides all or a portion of the respiratory gases for the subject by providing inhalation gases in inhalation limb 5. The inhalation gases pass through Y-connector 9 and into patient limb 10 for supply to the subject On exhalation, the respiratory gases pass through patient limb 10, Y-connector 9, and exhalation limb 7 back to ventilator 3.

As shown in Fig. 1, the nebulizer apparatus 1 is preferably positioned in patient breathing circuit 2 as near the subject as possible to minimize the aerosol transport path, and to minimize the deposition of the aerosol on the walls of the breathing circuit. To this end, nebulizer apparatus 1 may be inserted in the breathing circuit between Y-connector 9 and patient limb 10. The Y-connector 9 has a socket 11 for receiving tubular projection 12 of adapter 13 for nebulizer apparatus 1. The tubular socket 14 of the adapter 13 receives the patient limb 10. The adapter 13 also has a tubular socket 15 in which nebulizer apparatus 1 is placed. Nebulizer apparatus 1 has tubular projection 16 for this purpose. When nebulizer apparatus 1 is not needed, or when the nebulizer apparatus is removed for cleaning or maintenance, a cap (not shown) may be fitted into or over the opening of socket 15 to allow breathing circuit 2 to function in a normal manner. Alternatively, the entire adapter 13 containing nebulizer apparatus 1 may be removed from the breathing circuit and patient limb 10 reconnected directly to Y-connector 9. Control unit 4 is typically located separately from nebulizer apparatus 1 and may be incorporated in ventilator 3, if desired.

Nebulizer apparatus 1 comprises a removable liquid reservoir 17 for material to be nebulized. In the embodiment shown in the drawings, reservoir 17 is removably mounted on nebulizer apparatus 1. Alternatively it may be remote from nebulizer apparatus 1. A two-part, liquid controlling valve 18 is provided between the liquid reservoir 17 and the atomizing means 19 of nebulizer apparatus 1. One part of the valve is attached to removable reservoir 17 whereas the other part is placed inside the body of nebulizer 1. The two parts together form control valve 18. Electrical control signals are supplied to control valve 18 via cable 20 from control unit 4.

As noted above, the material to be nebulized can comprise an aqueous solution, or a particulate or colloidal suspension, of a product, such as a pharmaceutical agent. For purposes of explanation, the material undergoing nebulization is hereinafter generally described as a liquid.

Nebulizer apparatus 1 is shown in detail in the cross sectional, exploded view of Fig. 2. Nebulizer apparatus 1 shown in Fig. 2 has an annular housing 22 with the tubular projection 16 in the lower part of it that mounts the apparatus in socket 15 of adapter 13. Housing 22 is formed of plastic or similar material. The planar base member 23 formed on the lower edge of housing 22 contains circumferentially spaced projecting parts 24 and 25. Housing 22 is attached to internal plug member 30 by a spiral or bayonet fastening formed, in part, by openings 26 and 27. Associated projections 31 and 32 are situated symmetrically on opposite sides of plug member 30 and fit into openings 26 and 27 formed in the housing 22. Plug member 30 may be separated from, or joined to, housing 22 by turning and pulling or turning and pushing plug member 30 with respect to housing 22. This allows the portions of apparatus 1 carrying out the nebulizing of the liquid, positioned in cavity 28 of housing 22, to be removed at the end of therapy for replacement, or for cleaning when a different pharmaceutical agent is to be administered to the subject.

Projecting parts 24 and 25 separate the disc-like plate 50 from planar base member 23, preventing the opposing faces of the elements from touching. Plate 50 is shown with enlarged thickness in Fig. 2. The diameter of plate 50 is smaller than the diameter of cavity 28. Plate 50, made of a conductive material such as brass, contains a central opening 51. A mesh plate 52, having holes 53, is attached to plate 50 in central opening 51. Mesh plate 52 may be mounted to plate 50 by gluing with glue, brazing, welding, or other suitable technique. Plate 50 and mesh plate 52 may be formed from a single sheet of material, if desired.

Mesh plate 52 is relatively thin plate having a plurality of holes 53. Mesh plate 52 may be about 0.02 mm thick. The diameter of the holes at front surface 54 is preferably approximately 2-10 µm in diameter. Such holes may be formed in the plate by an electroforming process, which process produces holes of increasing diameter toward rear surface 55. However, straight holes will work equally well, the primary criterion being that the exit diameter in front surface 54 of mesh plate 52 is such as to form droplets of the desired size.

Front surface 54 of mesh plate 52 is exposed to the pressure of the breathing gases in breathing circuit 2. These pressures will vary during inhalation and exhalation conditions in breathing circuit 2. For example, with artificial ventilation, breathing circuit pressures may increase up to 100 mbar during inspiration and thereafter decrease during expiration. Projecting parts 24 and 25 separating the disc-like plate 50 from the planar member 23 form a pressure balancing channel between the plate 50 and planar member 23 together with the spacing between the plate 50 and the inside of the side wall of housing 22. These channels connect the volume between plug member 30 and plate 50 with breathing circuit 2 for equalizing the prevailing pressure at both sides of mesh plate 52. The pressure balancing provided by the channels prevents breathing gas from flowing through the holes in mesh plate 52, in opposition to the liquid being nebulized, which flow might otherwise degrade the operation of nebulizer apparatus 1. It also avoids pressure stressing of mesh plate 52 and causing leaks to occur through the mesh plate.

A ring-like vibrating element, such as a piezoelectric element 56, is mounted adjacent the upper surface of plate 50. Specifically, the piezoelectric element 56 is spaced from plate 50 by a small gap 57 and secured to plate 50 about its periphery by a conductive glue, brazing, welding, or other suitable technique, shown as 58 in Fig. 2. Piezoelectric element 56 has a central opening coaxial with the central opening 51 of plate 50.

Plug member 30 is formed from a non-conductive material, such as plastic, and is placed in the cavity 28 defined by the housing 22. Lower part of plug member 30 has two power terminals 35 and 36 made of conductive material. Plug member 30 is placed on top of plate 50 so that the plate is between the electrodes and projecting parts 24 and 25. The terminal 35, which terminal may be in the form of a ring- shaped spring, contacts piezoelectric element 56 and is also connected to cable 20 through the plug member 30. A second electrical power terminal 36 is also connected to cable 20 through plug member 30 and contacts conductive plate 50. Terminal 36 may be electrically grounded for purposes of applying a voltage to piezoelectric element 56 in conjunction with terminal 35.

Tubular sensing electrode 38, which is close to the upper surface of mesh plate 52, it is used for impedance measurement of the presence of liquid in nebulizer apparatus 1 in conjunction with plate 50 which is opposite the sensing electrode. A small domed cavity 37 may be formed in the lower surface of plug member 30 to surround sensing electrode 38.

Valve 18, which is used for controlling the supply of the liquid to vibrating mesh plate 52, is comprised of two components including a fixed part 39 and a changeable or disposable part 69. The fixed part 39 is located inside plug member 30 and the disposable part 69 is affixed to liquid reservoir 60. The disposable part 69 is designed in a tubular form to extend through the cavity 40 of the fixed part 39, when the reservoir is attached to plug member 30. The disposable part 69 is capable, by itself, of functioning as a shut off for the reservoir, but, in conjunction with fixed part 39, it also functions as a valve which is used to regulate the fluid flow from the reservoir to vibrating mesh plate 52. The valve, which is used for controlling the supply of liquid to vibrating mesh plate 52, is described in more detail below.

Reservoir 17, identified more particularly as 60 in Figs. 2 et seq., is attached to the top of plug member 30 by spiral or bayonet fastening formed by openings 61 and 62. Associated projections 33 and 34 are situated symmetrically on the sides of plug member 30 and fit into openings 61 and 62 formed in the reservoir 60. Reservoir 60 may be fastened to, or unfastened from, plug member 30 by turning and pushing or turning and pulling the reservoir with respect to plug member 30. This allows the reservoir to be removed at the end of therapy for replacement, or when a different drug is to be administered to the subject.

Reservoir 60 comprises a peripheral member 63 and a plate 64 made of material such as plastic, an elastic membrane 65 made of a material such as rubber, and the changeable or disposable part 69 of valve 18 affixed to the bottom of plate 64. Membrane 65 is stretched on the top of plate 64, which is then pushed into peripheral member 63. Membrane 65, which is between plate 64 and peripheral member 63, is locked in the channel 66 by the plate 64. Reservoir 60 may alternatively be formed as a single part by using the modern multi component fabrication techniques using plastics.

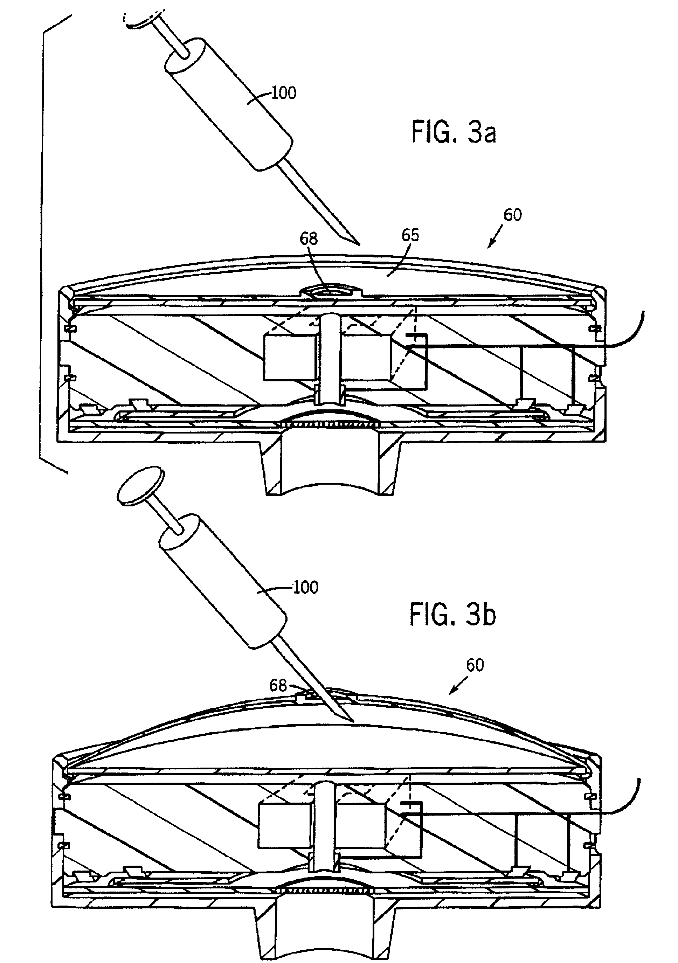

In the middle of membrane 65, on the upper side, is a thicker area 67 of membrane separated by ring 68. Reservoir 60 is filled with a liquid to be atomized by injecting the liquid with a syringe 100 through area 67 inside the ring 68. Specifically, when the reservoir is empty, membrane 65 is in the pre-strained state shown in Fig. 3a, which establishes enough pressure to empty the space beneath the membrane of liquid inside the reservoir. When the reservoir is filled with a liquid by injecting it with a syringe through the membrane in the area 67, as shown in Fig. 3b, pressure inside the reservoir increases as the membrane stretches.

In operation, valve 18, which is used for supplying the liquid to vibrating mesh plate 52, is opened in response to a signal from cable 20 and liquid flows from reservoir 60 toward the mesh plate 52. The liquid flows out through the disposable part 69, which is placed inside the cavity of fixed part 39 and tubular sensing electrode 38, into contact with the upper surface of mesh plate 52. The cohesive forces in the liquid create a column of liquid extending between the lower end of sensing electrode 38 and the mesh plate 52. To control the transport of liquid from reservoir 60 to mesh plate 52, the sensing electrode 38 and the mesh plate 52 function as sensing electrodes that detect the presence of liquid between the lower end of sensing electrode 38 and the rear surface 55 of mesh plate 52. The detection is based on alteration of the impedance between the two elements that function as sensing electrodes.

With the continued supply of liquid, the increased column of liquid formed between the end of sensing electrode 38 and the rear surface 55 of mesh plate 52 will significantly alter this impedance. A signal from mesh plate 52 is obtained via terminal 36 and a signal from sensing electrode 38 is obtained via conductor 100. The signals from the two sensing electrodes are inputted to an impedance sensor inside the control unit 4 via cable 20 and are used by the control unit 4 to close valve 18 to terminate or reduce the supply of liquid. When the impedance between the electrodes changes due to the liquid receding away from the end of sensing electrode 38 and the rear surface 55 of mesh plate 52 during operation of nebulizer apparatus 1, the valve opens again to allow the flow of liquid from the reservoir. The delivery of liquid to be nebulized can be controlled either by continuously vibrating mesh plate 52 and continuously regulating the liquid supply or by regulating the activation of mesh plate vibration and intermittently supplying liquid when the amount of liquid between the mesh plate 52 and the end of sensing electrode 38 is reduced.

High frequency voltage is supplied from a power source inside the control unit 4 though cable 20 and terminals 35 and 36 to piezoelectric vibrating element 56 to cause the element to vibrate. The voltage causes the element 56 to contract from the normal condition, shown in Fig. 4a, to a radially decreased condition shown in Fig. 4b and return to the normal condition. Due to the joinder of piezoelectric element 56 to plate 50 about the periphery of the element, the radial size reduction of piezoelectric element 56 causes plate 50 to deflect or displace, as by bowing, as shown in Fig. 4b, and then return to the flat condition, shown in Fig. 4a, when piezoelectric element 56 returns to the normal state. The action of plate 50 shown in Figs. 4a and 4b discharges nebulized liquid from holes 53 in mesh plate 52. At the front surface 54 of the vibrating mesh plate 52, the atomized liquid will grow into drops at each hole 53 due to the liquid surface tension. The drops will increase in size until the expelling forces arising from the movement of mesh plate 52 and the mass of each drop exceeds the holding force determined by the size of the holes 53 in mesh plate 52 and the surface tension of the liquid. The drops expelled from plate 52 pass through tubular projection 16 housing 22 into the patient limb 10 are inhaled by the subject as nebulized liquid.

As noted above, the valve 18, which is used for controlling the liquid supply from reservoir 60 to vibrating mesh plate 52, is formed of a fixed part 39 and a changeable or disposable part 69. The fixed part 39 is located inside plug member 30 and the disposable part 69 is affixed to liquid reservoir 60. The disposable part 69 includes elements that regulate the liquid flow through the valve under the influence of a magnetic field, which is produced with the fixed part 39. As noted above, the disposable part 69 by itself functions as a shut off, preventing liquid flow from the reservoir 60. Disposable part 69, together with fixed part 39, functions as an electrically controlled flow valve. Disposable part 69 and fixed part 39 of the valve are shown in more detail in Figs. 5 through 9.

Disposable part 69 comprises a tube 82, which is affixed to the bottom of plate 64 of reservoir 61. See Figs. 2 and 7. Or, tube 82 may be integral with plate 64. Tube 82 is made of an elastic material such as silicone or plastic. A stopper 83 is placed inside the tube 82. Stopper 83 is made of a material that changes shape when a magnetic field is applied to it. Two examples of stopper 83 are shown in more detail in Fig. 5a.

One example of stopper 83 is shown in Figs. 5a-1 and 5a-2. Stopper 83 there shown may be cylindrical in form. Stopper 83 has a lengthwise channel 84 going into the stopper from the upper surface of the stopper. The lower end of channel 84 branches out to smaller transverse channels 85. The transverse channels are located near in the middle of the stopper. Other transverse channels 86, beneath channels 85, extend into stopper 83 to join to a lengthwise channel 87 opening at the bottom surface of stopper 83.

Another example of stopper 83 is shown in Figs. 5a-3 and 5a-4. The stopper 83 shown in these figures is flat and somewhat plate-like in form having rounded edges. Near at the both ends of the stopper there are transverse channels 88 opening on the flat sides of the stopper.

Fixed part 39 may comprise an electromagnet constructed as shown in Figs. 2 and 5b. The electromagnet comprises a ferromagnetic body or armature 90 generally U-formed in shape and a coil 91, wound around all or a portion of the body. In Fig. 5b the coil is shown as extending over one side of the ferromagnetic body 90. Coil 91 is connected to cable 20 via appropriate power supply connectors 91a. The space 95 between jaws 92 and 93 of ferromagnetic body 90 may form the cylindrical cavity 40 when the electromagnetic is embedded in plug member 30 as shown in cross sectional exploded view in Fig 2. Tube 82 of disposable part 69 fits in the cavity 40 when reservoir 60 is attached to plug member 30 so that stopper 83 is positioned between the jaws 92 and 93 of the electromagnet. The sensing electrode 38 for impedance measurement may be formed as a ring which surrounds tube 82. The lower end of tube 82 is approximately the height of the lower end of sensing electrode 38.

An alternative embodiment for fixed part 39 is shown in Fig. 5c. In this embodiment, fixed part 39 consists of a cylindrical ferromagnetic body 152 and a coil 153 in body 152. Coil 153 is connected to cable 20 via appropriate power supply connectors 153a. The tubular cavity 151 is formed inside coil 153 and extends through it. Tube 82 of disposable part 69 fits in the cavity 151 when reservoir 60 is attached to plug member 30 so that stopper 83 is positioned in the middle of cavity 151 inside coil 153.

U.S. Patent 5,958,154 describes magnetically-controlled shape memory materials that change shape in a magnetic field. Such materials are often called magneto-shape-memory (MSM) alloys and have found use in actuators, sensors and other applications. These materials are analogous to conventional shape memory materials, but with the main difference that a MSM material is characterized by a magnetocrystalline anisotropy energy that is sufficient for enabling motion of twin boundaries of the martensitic twinned crystal structure in response to application of a magnetic field to the martensitic twinned crystal structure. This enables the material to change dimensions and to produce an actuation action in response to the magnetic field. Conversely, the magnetic field around such a material is altered when proportions of the variants are changed by mechanical loading. Results of over 5 percent reversible linear strains and bending strains as high as 15 percent have been obtained with magneto-shape-memory material. Such materials are made and sold by Adaptive Materials Technology Oy of Finland.

Variants, or twin variants, are regions in a crystal that have different crystallographic orientations while at the same time preserving the crystal lattice coherency at the interfaces, called twin boundaries, between different variants. A mechanism based on the magnetic-field-induced reorientation of the twin structure of the material in a macroscopic sense is shown in Figs. 6a and 6b, which illustrate a two-dimensional detail of the martensitic twinning structure. The applied magnetic field controls the reorientation of the twin variants in a way analogous to the way in which the twin variants are controlled by stress in conventional shape memory alloys, but in this mechanism, magnetostrictive strains have no role. In crystalline ferromagnetic materials, magnetization vectors lie along certain definite crystallographic axes called directions of easy magnetization, which is shown in Fig. 6a. Crystal anisotropy energy is an energy that directs the magnetization along these directions. When an external magnetic field is applied, as in Fig. 6b, the magnetization tends to turn from the easy direction for the unit cell to the direction of the external magnetic field as shown on the right side in Fig. 6b. The magnetic field thereby controls reorientation of martensitic twin variants such that the twin variants or variants in a favorable orientation relative to the field orientation grow at the expense of other twins resulting in a shape change of the material, which can be used to effect an actuation. A return actuation, corresponding to a recovery of the original material shape and corresponding original twin variant orientation, is obtained by removal of the applied magnetic field or by reorientation of the applied magnetic field to another direction.

If the magnetic straining of an MSM material is opposed by a high mechanical stress, the magnetically oriented twin structure could be recovered to the original structure under the stress, and the dimensions of the material that it had before magnetic straining are returned. The magnetization may, however, still remain in the direction of the external field under load. As the external stress is removed, the twin variants turn again in such a way that their easy direction of magnetization is close to the external field. At the same time, the dimensions of the material that existed before the mechanical stress was applied are recovered.

Magnetic control of the shape memory effect is possible in several ferromagnetic materials in which martensite interfaces or twin boundaries are glissile and magnetic anisotropy energy is high. In MSM actuators, the magnetic field is typically perpendicular to the long dimension of the actuating MSM element, but with the right orientation of lattice it may be controlled with a lengthwise magnetic field. The magnetic - field induced shape changes of the actuating elements are extension, bending, and torsion.

Functioning of the valve, i.e. to supply the liquid from the reservoir to the vibrating mesh plate, composed of disposable part 69 attached to fixed part 39 is shown in more detail in Figs. 7, 8, and 9. Figs. 7a and 7b are schematic views of the valve having a stopper of the type shown in Figs. 5a-1 and 5a-2 tightly placed inside elastic tube 82 of disposable part 69. Channels 84 and 87 are shown as enlarged over the showing in Fig. 5a. In the rest state in which coil 91 is not connected to a voltage source and there is no applied magnetic field, stopper 83 prevents fluid flow from the reservoir through the valve, as shown in Fig. 7a. When the coil 91 is connected to a voltage source, the current in the coil produces a magnetic field, which is directed through ferromagnetic body 90 between the jaws 92 and 93 as shown in the lower view in Fig. 7b. The stopper 83 inside tube 82 bends in the applied magnetic field between the jaws 92 and 93. The bending of stopper 83 causes the elastic tube 82 to expand on one side whereas on the opposite side a cavity arises between the walls of tube and stopper. A path for fluid flow is opened from inlet channel 84 to outlet channel 87 via a pair of crosswise channels 85 and 86 in the space on the one side between the walls of tube and stopper, as shown in Fig. 7b. When the applied magnetic field is removed, by disconnecting the voltage to coil 91, the stopper returns, by straightening, to its residual state shown in Fig. 7a. The walls of elastic tube 82 return to their original condition, abutting the walls of stopper, which causes the liquid flow path from inlet channel 84 to outlet channel 87 via the pair of crosswise channels 85 and 86 to close.

Figs. 8a and 8b are schematic views of the valve having a stopper shown in Fig. 5a-3 and 5a-4 placed inside the tube 82 of disposable part 69. In the rest state, shown in Fig. 8a, in which coil 91 is not connected to a voltage source and there is no applied magnetic field, stopper 83 prevents the fluid to flow from the reservoir through the valve, as shown in Fig. 8a. When the coil 91 is connected to a voltage source as shown in Fig. 8b, the current produces a magnetic field, which is directed through the ferromagnetic body 90 between the jaws 92 and 93. See Fig. 8b. The stopper 83 inside tube 82 bends in the applied magnetic field between the jaws 92 and 93. The bending of stopper 83 causes the elastic tube 82 to expand on one side whereas on the opposite side, a cavity arises between the walls of the tube and stopper. A path for fluid flow is opened from inlet opening 88 to outlet opening 89 in stopper 83 via the space between the walls of tube and stopper. When the applied magnetic field is removed by disconnecting the voltage to coil 91, the stopper returns to its rest state by straightening. The walls of clastic tube 82 resume their original condition, abutting the walls of stopper 83, which causes the path from opening 88 to opening 89 to close.

While Figs. 7 and 8 show embodiments of a valve in which a normally closed valve is opened by the application of a magnetic field, it will be appreciated that the valve may be so constructed that a normally open valve is closed by the application of a magnetic field. For example, stopper 83 could be formed in the bent shape shown in Fig. 7b to provide a normally open valve. The application of a magnetic field would straighten stopper 83 to the condition shown in Fig. 7a to close the valve.

A further embodiment of the valve is shown in Figs. 9a and 9b. As shown in Fig. 9b, the jaws 201 and 202 of a valve member or stopper 200 bend and open in an applied magnetic field produced with an electromagnet. When the jaws are open, liquid flows between the jaws and through the cavity 203 and the valve is open. The liquid flow is stopped when jaws 201 and 202 are brought together, as turning off the applied magnetic field. See Fig. 9a. Stopper 200 may be mounted in an elastic tube to form the valve.

Figs. 10a and 10b and 11a and 11b show another embodiment of a valve of the present invention suitable for use in a liquid discharge apparatus. In this embodiment, a valve is provided internally of liquid reservoir 60. To this end, valve disk 300 is placed over opening 302 in plate 64 of reservoir 60. Means are provided to bias valve disk 300 into contact with plate 64 to close opening 302. Such means may comprise a spring in a cage mounted on plate 64 or elastic threads 304 fastened to plate 64 and arranged in a cross over valve disk 300. The pressure of the liquid when reservoir 60 is filled also serves to bias the disk into contact with plate 64 and close opening 302. Tube 306 extends from opening 302 in plate 64.

An elastic area is provided in plate 64. The elastic area 308 is defined by ring shaped channel 310 having a smaller thickness than plate 64. The elastic area 308 can be formed in plate 64 by multi component fabrication techniques using, for example, plastics and rubber.

Actuator member 312, formed of a material that changes shape when subjected to a magnetic field, is loosely placed in a hole in plug member 30 between jaws 314 of armature 316. Fig. 10b shows the positioning of actuator 312 between jaws 314. The member abuts, and may be fastened to, the bottom of the hole. When subjected to a magnetic field, actuator member 312 changes shape from a residual shape to a raised position in which member 312 increases in height lengthwise. Projection 318 of actuator member 312 extends through hole 320 plug member 30. Coil 91 is wound around armature 316 to generate a magnetic field, when energized.

When liquid reservoir 60 is attached to plug member 30, the upper end of projection 318 is proximate to, but spaced from, elastic area 308. When coil 91 is energized, actuator member 312 increases in height from the residual state shown in Fig. 11a to the extended position shown in Fig. 11b. Projection 318 extends through hole 320 and presses elastic area 308. Elastic area 308 moves upwardly and lifts valve disk 300 off plate 64, allowing liquid in reservoir 60 to flow out opening 302 through tube 306 and into apparatus 1.

It is obvious that the liquid discharge apparatus and valve structure presented above can be applied to many applications and the construction of the apparatus and valve may be accomplished in many variations. It is thus recognized that other equivalents, alternatives, and modifications aside from those expressly stated, are possible and within the scope of the appended claims.

## Claims

1. An apparatus for discharging liquid comprising:
a housing having an opening through which the liquid is discharged;
a displaceable element in said housing which is subject to displacement for discharging liquid;
a means for displacing said element to cause liquid to be discharged from said opening;
a liquid reservoir; and
a valve for providing liquid from said reservoir to said displaceable element, said valve comprising,
a magnetic field source for selectively forming a magnetic field,
a flow controlling member coupled to said reservoir, said flow controlling member having,
a tube through which liquid may flow from said reservoir to said displaceable element, said tube lying in the magnetic field of said magnetic field source, when formed; and
a stopper in said tube formed of a material that changes from a residual shape to a deformed shape when subjected to a magnetic field, said stopper having a flow channel configuration which is sealed by said tube when said stopper is in one of the residual shape or deformed shape to prevent liquid flow from said reservoir to said displaceable element, said flow channel configuration being opened to form a liquid flow path in said tube when said stopper is placed in the other of the deformed or residual shape by subjection to the magnetic field of said magnetic field source to allow liquid to flow from said reservoir to said displaceable element for discharge from said apparatus.

2. An apparatus according to claim 1 wherein said stopper is further defined as having an axis generally parallel to said tube, said stopper having a top facing said reservoir, a bottom facing said displaceable element and a peripheral surface extending between said top and bottom in contiguity with said tube, said stopper having a pair of flow channels lying transverse to said axis and spaced along said axis, said flow channels opening on said peripheral surface of said stopper, said stopper being bent along said axis when subjected to the magnetic field.

3. An apparatus according to claim 2 wherein said stopper has a flow channel generally parallel to said axis of said stopper extending between said top of said stopper and a transverse flow channel, said stopper having a further flow channel generally parallel to said axis of said stopper extending between said bottom of said stopper and another transverse flow channel.

4. An apparatus according to claims 1, 2, or 3 wherein said flow channel configuration is sealed when said stopper is in the residual condition and open when said stopper is in the deformed condition.

5. An apparatus according to claim 1 wherein said stopper has an axis generally parallel to said tube, said stopper having a channel extending partially through said stopper parallel to said axis, said stopper having bifurcated closure member blocking said channel when said stopper is in one of the residual shape or deformed shape, said bifurcated closure members being spread apart in a direction transverse to the axis of said stopper when the stopper is in the other of the deformed shape or residual shape to allow liquid to flow from said reservoir to said displaceable element.

6. An apparatus according to claim 1 wherein said stopper is formed of a magneto-shape-memory material.

7. An apparatus according to claim 1, 2, 3 or 5 wherein said tube is elastic.

8. An apparatus for discharging liquid comprising:
a housing having an opening through which the liquid is discharged;
a displaceable element in said housing which is subject to displacement for discharging liquid;
means for displacing said element to cause liquid to be discharged from said opening;
a liquid reservoir mountable on said housing; and
a valve mounted on said reservoir for controlling the flow of liquid from said reservoir to said displaceable element along a flow path for the liquid;
a magnetic field source for selectively forming a magnetic field,
an actuator for said valve formed of a material that changes from a residual shape to a deformed shape when subjected to a magnetic field, said actuator causing said valve to assume one liquid flow controlling condition when said actuator is in the residual shape to prevent liquid flow from said reservoir to said displaceable element, said actuator causing said valve to assume another liquid flow controlling condition when said actuator is placed in the deformed shape by subjection to the magnetic field of said magnetic field source to allow liquid to flow from said reservoir to said displaceable element for discharge from said apparatus.

9. An apparatus according to claim 8 wherein said actuator is formed of a magneto-shape-memory material.

10. An apparatus according to claim 1 or 8 wherein said magnetic field source comprises a coil.

11. An apparatus according to claim 1 or 8 wherein said displaceable element is conductive and wherein said apparatus has further conductive means spaced from said displaceable element for measuring, in conjunction with said displaceable element, an impedance indicative of the amount of liquid provided to said displaceable element.

12. An apparatus according to claim 11 wherein said conductive means is proximate an end of said tube.

13. An apparatus according to claim 1 or 8 wherein said liquid reservoir is removably attachable to said apparatus.

14. An apparatus according to claim 13 wherein said reservoir is formed to pressurize the liquid therein.

15. An apparatus according to claim 1 or 8 wherein said displacing means comprises piezoelectric means.

16. An apparatus according to claim 1 or 8 wherein said displacing means comprises a piezoelectric vibrator.

17. An apparatus according to claim 1 or 8 wherein said displaceable element is subject to bowing to create the displacement.

18. An apparatus according to claim 16 wherein said displaceable means include a mesh plate for atomizing the liquid.

19. An apparatus according to claim 11 further including means coupled to said apparatus for energizing said magnetic field source responsive to the impedance measurement.

20. An apparatus according to claim 1, 2, 3, 5, 6, or 8 further defined as a nebulizer for discharging atomized liquid.

21. A fluid flow control valve comprising:
a magnetic field source for selectively forming a magnetic field;
an elastic tube through which fluid may flow, said tube lying in the magnetic field of said magnetic field source, when formed; and
a stopper in said tube, said stopper being formed of a material that changes from a residual shape to a deformed shape when subjected to a magnetic field, said stopper having a flow channel configuration which is sealed by said tube when said stopper is in one of the residual shape or deformed shape to prevent fluid flow through said tube, said flow channel configuration being opened to form a fluid flow path in said tube when the stopper is placed in the other of the deformed shape or residual shape by subjection to the magnetic field of said magnetic field source.

22. A fluid flow control valve according to claim 21 wherein said stopper is further defined as having an axis generally parallel to said tube, said stopper having a top facing one end of the tube, a bottom facing another end of said tube, and a peripheral surface extending between said top and bottom in contiguity with said tube, said stopper having a pair of flow channels lying transverse to said axis and spaced along said axis, said flow channels opening on the peripheral surface of said stopper, said stopper being bent along said axis when subjected to the magnetic field.

23. A fluid flow control valve according to claim 22 wherein said stopper has a flow channel generally parallel to the axis of said stopper extending between said top of said stopper and a transverse flow channel, said stopper having a further flow channel generally parallel to said axis of said stopper extending between said bottom of said stopper and another transverse flow channel.

24. A fluid flow control valve according to claims 21, 22, or 23 wherein said flow channel configuration is sealed when said stopper is in the residual condition and wherein said flow channel is open when said stopper is in the deformed condition.

25. A fluid flow control valve according to claim 21 wherein said stopper has an axis generally parallel to said tube, said stopper having a channel extending partially through said stopper parallel to said axis, said stopper having a bifurcated closure member blocking said channel when said stopper is in one of the residual shape or deformed shape, said bifurcated closure members being spread apart in a direction transverse to the axis of said stopper when said stopper is in the other of said deformed shape or residual shape to allow fluid to flow through said tube.

26. A fluid flow control valve comprising:
a magnetic field source for selectively forming a magnetic field;
fluid flow path means through which the fluid may flow;
a valve for controlling the flow of fluid through said fluid flow path means; and
an actuator for said valve formed of a material that changes from a residual shape to a deformed shape when subjected to a magnetic field, said actuator causing said valve to assume one fluid flow controlling condition when said actuator is in the residual shape, said actuator causing said valve to assume another fluid flow controlling condition when said actuator is placed in the deformed shape by subjection to the magnetic field of said magnetic field source.

27. A fluid flow control valve according to claim 21 or 26 wherein said stopper is formed of a magneto-shape-memory material.

28. A fluid flow control valve according to claim 21 or 26 wherein said magnetic field source comprises a coil.

29. A fluid flow control valve according to claims 21 or 26 wherein said stopper or actuator opens said valve when subjected to a magnetic field.
